(19) Europäisches Patentamt
European Patent Office
Office européen des brevets

(11) Veröffentlichungsnummer: **0 252 009 B1**

(12) # EUROPÄISCHE PATENTSCHRIFT

(45) Veröffentlichungstag der Patentschrift: **01.08.90**

(51) Int. Cl.[5]: **C07C 317/14,** D06L 3/12, C07C 317/24, C07C 47/55

(21) Anmeldenummer: **87810362.1**

(22) Anmeldetag: **25.06.87**

(54) **1,4-Distyrylbenzolverbindungen und deren Mischungen mit anderen 1,4-Distyrylbenzolverbindungen.**

(30) Priorität: **01.07.86 CH 2637/86**

(43) Veröffentlichungstag der Anmeldung: **07.01.88 Patentblatt 88/1**

(45) Bekanntmachung des Hinweises auf die Patenterteilung: **01.08.90 Patentblatt 90/31**

(84) Benannte Vertragsstaaten: **CH DE FR GB IT LI**

(56) Entgegenhaltungen:
**EP-A- 0 030 917**
**EP-A- 0 032 254**
**EP-A- 0 033 018**
**DE-A- 1 794 386**
**DE-A- 2 401 665**
**DE-A- 3 339 383**

(73) Patentinhaber: **CIBA-GEIGY AG, Klybeckstrasse 141, CH-4002 Basel(CH)**

(72) Erfinder: **Meyer, Hans Rudolf, Dr., Bollwerkstrasse 102, CH-4102 Binningen(CH)**
Erfinder: **Guglielmetti, Leonardo, Dr., Wartenbergstrasse 6, CH-4103 Bottmingen(CH)**

**EP 0 252 009 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung sind neue asymmetrisch substituierte 1,4-Distyrylbenzolverbindungen, deren Mischungen mit symmetrisch substituierten 1,4-Distyrylbenzolverbindungen, Verfahren zur Herstellung der vorgenannten Verbindungen oder Mischungen und Mittel zum optischen Aufhellen von Polyesterfasern sowie neue, bei der Herstellung der vorgenannten Verbindungen intermediär gebildete Aldehyde.

Symmetrisch substituierte 1,4-Distyrylbenzolverbindungen und Verfahren zu deren Herstellung sind in den DE-A 112 072, DE-A 1 444 003 und DE-A 2 401 665 beschrieben, während asymmetrisch substituierte 1,4-Distyrylbenzolverbindungen und deren Mischungen mit asymmetrisch und/oder symmetrisch substituierten 1,4-Distyrylbenzolverbindungen sowie Verfahren zu deren Herstellung aus den EP-A 30 917, EP-A 32 254, DE-A 3 339 383 und DE-A 3 347 576 bereits bekannt sind. In der EP-A 33 018 werden Mischungen aus ortho-cyan-substituierten 1,4-Distyrylbenzolverbindugen mit substituierten Naphthalimiden beschrieben. Allen gemeinsam ist die Verwendung als optische Aufheller für insbesondere Polyesterfasern.

Die neuen asymmetrisch substituierten 1,4-Distyrylbenzolverbindungen sind durch die Formel

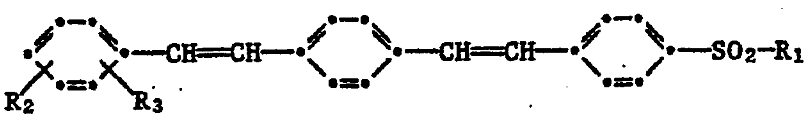

(1)

gekennzeichnet, worin $R_1$ $C_1$-$C_4$-Alkyl oder Phenol, $R_2$ Cyano, $COOC_1$-$C_4$-Alkyl oder Chlor und $R_3$ Wasserstoff oder Chlor bedeuten.

Die neuen Verbindungen der Formel 1 sowie deren Mischungen mit den zum Teil vorbekannten symmetrisch substituierten 1,4-Distyrylbenzolverbindungen der Formeln

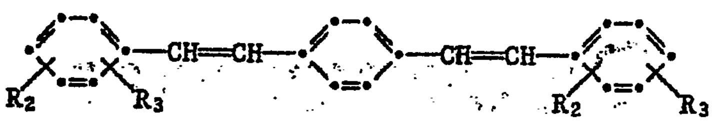

(2)

und/oder

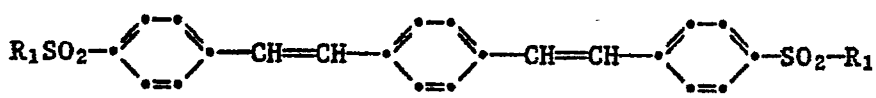

(3)

zeigen gegenüber den Einzelkomponenten der Formeln 2 und 3 als optische Aufheller von Polyesterfasern oder Polyesterfasern enthaltendem Material einen verbesserten Weissgrad, besonders bei niedriger Fixiertemperatur.

Bevorzugte 1,4-Distyrylbenzolverbindungen der Formel 1 sind solche, in denen $R_1$ Methyl oder Aethyl, $R_2$ Cyano oder 4-Chlor und $R_3$ Wasserstoff oder, falls $R_2$ = 4-Chlor ist, 3-Chlor bedeuten, und insbesondere solche, worin $R_1$ Methyl, $R_2$ 2-Cyano und $R_3$ Wasserstoff bedeuten.

Bevorzugte Mischungen von asymmetrischen mit symmetrischen 1,4-Distyrylbenzolverbindungen enthalten 1 bis 99 Gew.-% 1-(2-Cyanostyryl)-4(4-methylsulfonyl- oder 4-äthylsulfonyl-styryl)-benzol, 1 bis 99 Gew.-% 1,4-Di(2-cyanostyryl)-benzol und 0 bis 20 Gew.-% 1,4-Di(4-methylsulfonyl- oder 4-äthylsulfonyl-styryl)-benzol.

Gegenstand der vorliegenden Erfindung sind auch Mittel zum optischen Aufhellen von Polyesterfasern oder Polyesterfasern enthaltendem Material, welche mindestens eine 1,4-Distyrylbenzolverbindungen der Formel

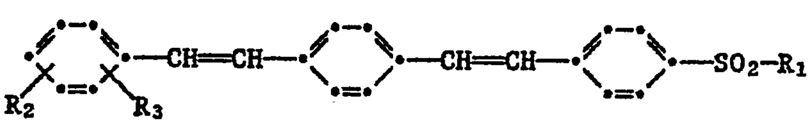

(1)

oder deren Mischung mit mindestens einer der Verbindungen der Formeln

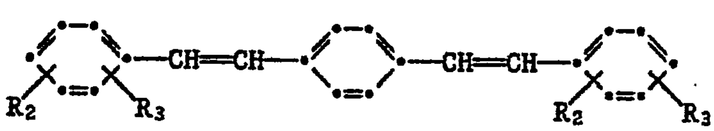

(2)

und/oder

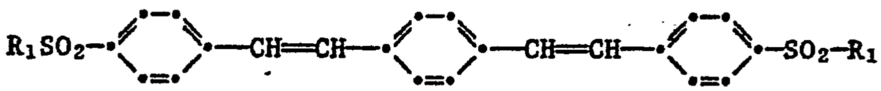

(3)

enthalten, wobei in den Verbindungen der Formel 1 bis 3 $R_1$ $C_1$-$C_4$-Alkyl oder Phenyl, $R_2$ Cyano, $COOC_1$-$C_4$-Alkyl oder Chlor und $R_3$ Wasserstoff oder Chlor bedeuten.

Die erfindungsgemässen Mittel können ausserdem noch eine oder mehrere an sich bekannte optische Aufheller aus der Reihe der 1,4-Distyrylbenzole, 4,4'-Distyryl-biphenyle, 2,5-Dibenzoxazolylthiophene, 1,2-Dibenzoxazolyl-äthylene, Mono- und Dibenzoxazolylstilbene oder -styrole, Triazinyl-pyrene, 4-Triazolylstilbene, Isoxazolyl-stilbene, Dibenzoxazolyl-naphthaline, Triazolyl-, Pyrazolyl- und Phenyl-cumarine, 4,4'-Divinyl-stilbene und Naphthalimide enthalten.

Die Herstellung der erfindungsgemässen asymmetrisch substituierten 1,4-Distyrylbenzolverbindungen der Formel 1 oder deren Mischungen mit symmetrisch substituierten 1,4-Distyrylbenzolverbindungen der Formeln 2 und/oder 3 erfolgt durch Kondensation von Terephthalaldehyd in einer oder zwei Reaktionsstufen mit den beiden Phosphonaten der Formeln

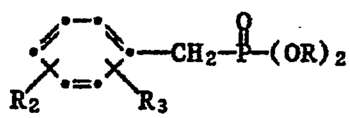

und

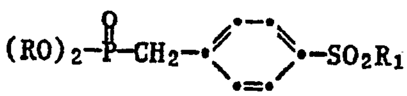

(5)

worin R $C_1$-$C_4$-Alkyl bedeutet.

Bei der Kondensation von Terephthalaldehyd mit den beiden Phosphonaten der Formeln 4 und 5 in zwei Reaktionsstufen kann man die intermediär gebildeten Aldehyde der Formeln

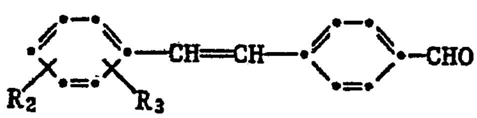

(6)

oder

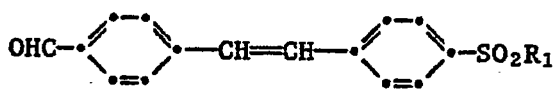

(7)

gegebenenfalls isolieren. Die als Zwischenprodukte isolierbaren Aldehyde der Formeln 7 und

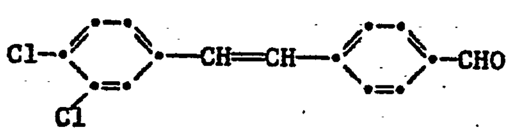

(8)

sind neu und werden somit als neue Verbindungen zusätzlich beansprucht. Während man bei der Herstellung der 1,4-Distyrylbenzolverbindungen der Formel 1 ein Mol Terephthalaldehyd mit jeweils einem Mol der beiden Phosphonate der Formeln 4 und 5 in 2 aufeinander folgenden Stufen kondensieren lässt, führt man bei der Herstellung von Mischungen der Verbindungen der Formel 1 mit Verbindungen der Formeln 2 und/oder 3 die Kondensation von Terephthalaldehyd mit den beiden Phosphonaten der Formeln 6 und 7 in den für die Mischungen jeweils vorgesehenen stöchiometrischen Mengenverhältnissen aus. Man kann die Mischungen in einfacher Weise auch dadurch herstellen, dass man die Verbindungen der Formel 1 mit denen der Formeln 2 und/oder 3 in Substanz mischt.

Die erfindungsgemässen Mittel eignen sich hervorragend zum optischen Aufhellen von Textilmaterialien aus linearen oder modifizierten Polyestern. Wie bei optischen Aufhellern üblich, können die einzelnen Verbindungen in einem flüssigen Medium, z.B. Wasser, dispergiert und dann die Dispersionen zusammengegeben werden. Man kann aber auch die aus dem obigen Verfahren erhältliche Mischung der Verbindungen der Formel 1 mit Verbindungen der Formeln 2 und/oder 3 bzw. eine Mischung dieser einzelnen Verbindungen in Substanz gemeinsam dispergieren, was in üblicher Weise in Kugelmühlen, Kolloidmühlen, Perlmühlen oder dergleichen geschieht. Die erfindungsgemässen Mittel können nach bekannten Methoden auf das Textilgut appliziert werden, beispielsweise nach dem Ausziehverfahren bei 90 bis 140°C oder nach dem Foulardthermverfahren bei 160 bis 220°C. Die Applikation erfolgt mit Vorteil in wässrigem Medium, worin die Verbindungen in feinverteilter Form als Suspensionen, sogenannten Mikrodispersionen, oder gegebenenfalls Lösungen vorliegen. Bei der Applikation können schliesslich noch Dispergier-, Stabilisier-, Netz- und weitere Hilfsmittel zugesetzt werden.

Die folgenden Beispiele veranschaulichen die Erfindung.

Beispiel 1

Zu einer Lösung von 9,5 g 4'-Methylsulfonyl-stilben-4-aldehyd und 9,5 g 2-Cyanobenzyl-diäthylphosphonat (Gehalt 97,2 %) in 60 ml Dimethylformamid tropft man bei Raumtemperatur 8,8 g 30%-ige methanolische Natriummethylatlösung. Man lässt 2 Stunden bei 40°C nachrühren, kühlt ab und versetzt mit 30 ml Wasser. Der blassgelbe Kristallbrei wird abgenutscht und wiederholt mit Methanol und Wasser gewaschen. Nach dem Trocknen im Vakuum erhält man 11,7 g der Verbindung der Formel

(101) 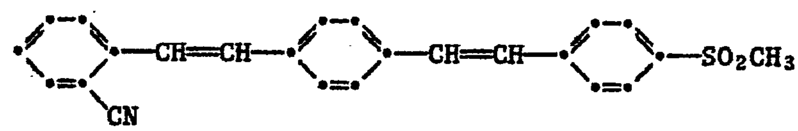

Smp. 215-216°C, nach Umkristallisation aus Chlorbenzol und Aethylenglykolmonomethyläther.

Den benötigten 4'-Methylsulfonyl-stilben-4-aldehyd erhält man wie folgt: Man trägt unter Rühren und Kühlen bei Raumtemperatur, sowie Ueberleiten von Stickstoff portionsweise 33,5 g Terephthalaldehyd in eine Lösung von 17,5 g Kaliumhydroxid (Gehalt 88 %) in 200 ml Methanol. Nachdem vollständige Lösung eingetreten ist, tropft man bei 10°C im Verlauf 1/2 Std. eine Lösung von 81,0 g p-Methylsulfonylbenzyl-diäthylphosphonat (Gehalt 94,5 g %, Smp. 66 - 68°C) hinzu, wobei das Reaktionsprodukt sich auszuscheiden beginnt. Man lässt die Suspension bei Raumtemperatur über Nacht ausrühren, kühlt im Eisbad ab, nutscht und wäscht den Rückstand wiederholt mit Methanol und Wasser. Nach dem Trocknen im Vakuum bei 80°C erhält man 65,7 g der Verbindung der Formel

(102) 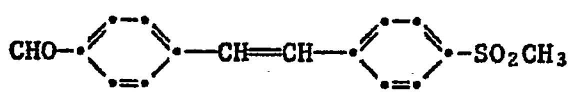

als blass-gelbes Pulver vom Smp. 188 - 190°C. Es lässt sich aus Xylol umkristallisieren.

Das p-Methylsulfonylbenzyl-diäthyl-phosphonat erhält man durch Erhitzen von p-Methylsulfonylbenzylchlorid mit überschüssigem Triäthylphosphit auf 140 - 145°C und anschliessende Destillation im Hochvakuum: Farbloses, dickflüssiges Oel, das in der Vorlage erstarrt, Smp. 70 - 72°C (umkristallisiert aus Tetrachlorkohlenstoff).

Beispiel 2

Zu einer Lösung von 2,7 g Terephthalaldehyd, 4,1 g 1-Diäthylphosphonomethyl-4-methylsulfonyl-benzol (Gehalt 75 %) und 7,8 g 2-Diäthylphosphonomethyl-benzonitril in 20 ml Dimethylformamid tropft man bei Raumtemperatur unter Rühren und Ueberleiten von Stickstoff, 7,9 g einer 30%-igen Lösung von Natriummethylat in Methanol. Man rührt noch 2 Stunden bei 30 - 40°C, verdünnt nach Abkühlen mit 20 ml Methanol und 10 ml Wasser und nutscht das ausgefallene Produkt ab. Nach wiederholtem Waschen mit Methanol und Wasser und Trocknen im Vakuum bei 80°C erhält man 6,0 g eines blass-gelben Pulvers bestehend aus 38 Gew.-% der Verbindung der Formel (101), 56 Gew.-% der Verbindung der Formel

(103) 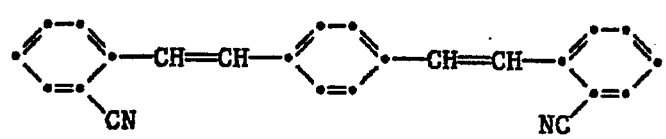

und 6 Gew.-% der Verbindung der Formel

(104) 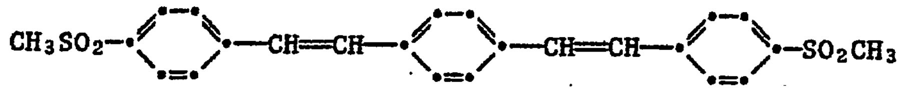

(Gehaltsbestimmung mittels High Pressure Liquid Chromatography)

<u>Beispiel 3</u>

Zu einer Lösung von 4,7 g der Verbindung der Formel

(105) 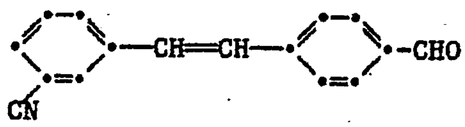

und 9,6 g p-Methylsulfonylbenzyl-diäthyl-phosphonat (Gehalt 70 %) in 60 ml Dimethylformamid tropft man bei Raumtemperatur 5,2 g einer 30%-igen methanolischen Natriummethylatlösung. Man rührt 2 Stunden bei 40 - 45°C, kühlt ab und versetzt mit 60 ml Wasser. Das ausgefallene Produkt wird abgenutscht, wiederholt mit Methanol und Wasser gewaschen und im Vakuum getrocknet. Man erhält 6,1 g der Verbindung der Formel

(106) 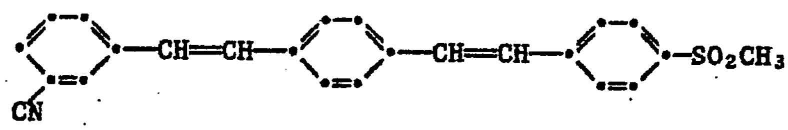

Smp. 226 - 227°C, nach Umkristallisieren aus Chlorbenzol und Aethylenglykolmonomethyläther.

<u>Beispiel 4</u>

Zu einer Lösung von 22,8 g der Verbindung der Formel

(107) 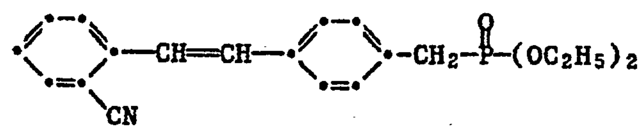

und 7,2 p-Methylsulfonyl-benzaldehyd in 100 ml Dimethylformamid fügt man 3,3 g Natriummethylat. Man rührt die Suspension 3 Stunden bei 45°C, lässt abkühlen und versetzt mit Wasser. Das ausgefallene Produkt wird abgenutscht und wiederholt mit Alkohol und Wasser gewaschen. Man erhält 12,0 g der Verbindung der Formel (101). Smp. 217 - 218°C (aus Chlorbenzol).

Die als Ausgangsprodukt benötigte Verbindung der Formel (107) erhält man z.B. durch Bromierung von 2-Cyano-4'-methyl-stilben mit N-Bromsuccinimid in siedendem Tetrachlorkohlenstoff in Gegenwart von Dibenzoylperoxid und anschliessende Umsetzung des erhaltenen 4-Brommethyl-2'-cyano-stilbens (Smp. 120 - 122°) mit überschüssigem Triäthylphosphit bei 140°C.

<u>Beispiel 5</u>

Verwendet man in Beispiel 1 anstelle von 2-Cyanobenzyl-diäthyl-phosphonat die entsprechende Menge 3,4-Dichlorbenzyl-diäthyl-phosphonat, das man bei 40°C zutropft, und arbeitet im übrigen wie beschrieben, so erhält man die Verbindung der Formel

(108) 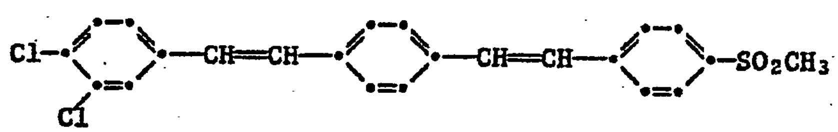

als blass-gelbe Kristalle von Smp. 264 - 265°C (umkristallisiert aus Chlorbenzol).

Man erhält die Verbindung der Formel (108) auch umgekehrt durch Kondensation des Aldehyds der Formel

(109) 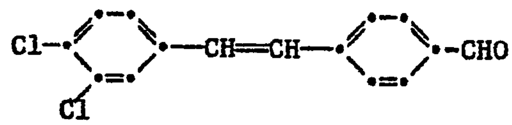

mit p-Methylsulfonylbenzyl-diäthyl-phosphonat gemäss Beispiel 1. Der Aldehyd der Formel (109) wird analog dem Aldehyd der Formel (102) hergestellt, wobei man anstelle von festem Kaliumhydroxid auch eine wässerige 50%-ige Lösung einsetzen kann. Zur Reinigung wird das Rohprodukt nach Heiss-klärfiltration aus Aethanol kristallisiert: Smp. 128 - 136°C.

Applikationsbeispiele

Beispiel 6

Ein Polyestergewebe (Terylene Typ 540) wird bei 40°C auf einem Färbeapparat bei einem Flottenverhältnis von 1 zu 20 mit einem wässrigen Bad, enthaltend 0,1 Gew.-% des optischen Aufhellers der Formel 101 (Beispiel 1) in fein dispergierter Form und 1 g/l eines Fettalkoholpolygykoläthers, behandelt. Innerhalb von 30 Minuten steigert man die Temperatur auf 130°C und belässt sie während weiterer 30 Minuten auf dieser Höhe. Dann kühlt man innerhalb von 15 Minuten wieder auf 40°C ab. Zur Nachbehandlung wird das Textilgut während 30 Sekunden in fliessendem deionisiertem Wasser gespült und bei 180°C getrocknet. Das derart behandelte Polyestergewebe weist einen hohen Aufhelleffekt auf.

Beispiel 7

Verwendet man nach der gleichen Vorschrift wie in Beispiel 6 anstelle des optischen Aufhellers der Formel 101 einen solchen der Formel 106 (Beispiel 3), so erhält man ähnlich gute Aufhelleffekte.

Beispiel 8

Verwendet man nach der gleichen Vorschrift wie in Beispiel 6 anstelle des optischen Aufhellers der Formel 101 die gleiche Menge eines optischen Aufhellers aus einer Zweiermischung von 70 Gew.-% der Verbindung der Formel 101 und 30 Gew.-% der Verbindung der Formel 103 (Beispiel 2), so erhält man ähnlich gute Aufhelleffekte.

Beispiel 9

Verwendet man nach der gleichen Vorschrift wie in Beispiel 6 anstelle des optischen Aufhellers der Formel 101 die gleiche Menge eines optischen Aufhellers aus einer Dreiermischung, deren Herstellung im Beispiel 2, beschrieben ist, bestehend aus 38 Gew.-% der Verbindung der Formel 101, 56 Gew.-% der Verbindung der Formel 103 und 6 Gew.-% der Verbindung der Formel 104, so erhält man ähnlich gute Aufhelleffekte.

Beispiel 10

Verwendet man nach der gleichen Vorschrift wie in Beispiel 6 anstelle des optischen Aufhellers der Formel 101 die gleiche Menge eines optischen Aufhellers aus einer Zweiermischung von 70 Gew.-% der Verbindung der Formel 108 (Beispiel 5) und 30 Gew.-% 1,4-Di-(3,4-dichlorstyryl)-benzol, so erhält man ähnlich gute Aufhelleffekte.

Beispiel 11

Verwendet man nach der gleichen Vorschrift wie in Beispiel 6 anstelle des optischen Aufhellers der Formel 101 die gleiche Menge eines optischen Aufhellers aus einer Dreiermischung von 32 Gew.-% der Verbindung der Formel 108 (Beispiel 5), 64 Gew.-% 1,4-Di-(3,4-dichlorstyryl)-benzol und 4 Gew.-% der Verbindung der Formel 104 (Beispiel 2), so erhält man ähnlich gute Aufhelleffekte.

Beispiel 12

Man foulardiert bei Raumtemperatur ein Polyestergewebe (Terylene Typ 540) mit einer wässrigen Flotte enthaltend 0,1 g/l des optischen Aufhellers der Formel 101 in dispergierter Form und 1 ml/l Invadin JFC 200 %. Der Abquetscheffekt beträgt 65 %. Anschliessend wird während 30 Minuten bei einer Temperatur von 80°C getrocknet und daraufhin bei 200°C thermofixiert. Das so behandelte Polyestergewebe weist einen hohen Aufhelleffekt auf.

Verwendet man nach der gleichen Vorschrift wie oben statt des optischen Aufhellers der Formel 101 einen solchen der Formel 108 (Beispiel 5) oder der Formel 108 (Beispiel 3) so erhält man ähnlich gute Aufhelleffekte.

Verwendet man nach der gleichen Vorschrift wie oben statt des optischen Aufhellers der Formel 101 die jeweils gleiche Menge an optischen Aufhellern aus den Zweier- und Dreiermischungen, wie sie zum Ausziehverfahren in den Beispielen 8 bis 11 genannt sind, so erhält man ähnlich gute Aufhelleffekte auch nach dem Foulardthermverfahren.

## Patentansprüche

1. 1,4-Distyrylbenzolverbindungen der Formel

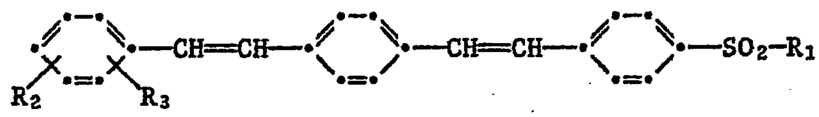
(1)

worin $R_1$ $C_1$-$C_4$-Alkyl oder Phenyl, $R_2$ Cyano, $COOC_1$-$C_4$-Alkyl oder Chlor und $R_3$ Wasserstoff oder Chlor bedeuten.

2. 1,4-Distyrylbenzolverbindungen gemäss Anspruch 1, wobei in Formel 1 $R_1$ Methyl oder Aethyl, $R_2$ Cyano oder 4-Chlor und $R_3$ Wasserstoff oder, falls $R_2$ = 4-Chlor ist, 3-Chlor bedeuten.

3. 1,4-Distyrylbenzolverbindungen gemäss Anspruch 1, wobei in Formel 1 $R_1$ Methyl, $R_2$ 2-Cyano und $R_3$ Wasserstoff bedeuten.

4. Mischungen von Verbindungen der Formel 1 gemäss Anspruch 1 mit Verbindungen der Formeln

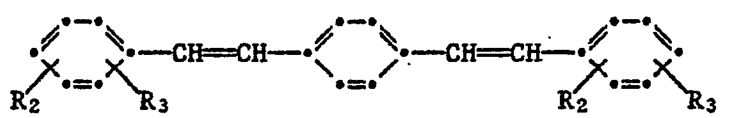
(2)

und/oder

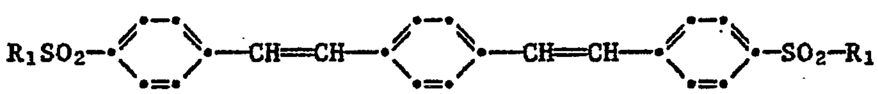
(3)

5. Mischungen gemäss Anspruch 4, enthaltend 1 bis 99 Gew.-% 1-(2-Cyanostyryl)-4-(4-methylsulfonyl- oder 4-äthylsulfonyl-styryl)-benzol, 1 bis 99 Gew.-% 1,4-Di(2-cyanostyryl)-benzol und 0 bis 20 Gew.-% 1,4-(Di(4-methylsulfonyl- oder 4-äthylsulfonyl)-benzol.

6. Mittel zum optischen Aufhellen von Polyesterfasern, enthaltend mindestens eine 1,4-Distyrylbenzolverbindung der Formel

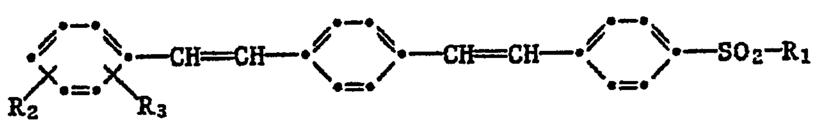
(1)

oder deren Mischungen mit mindestens einer Verbindung der Formeln

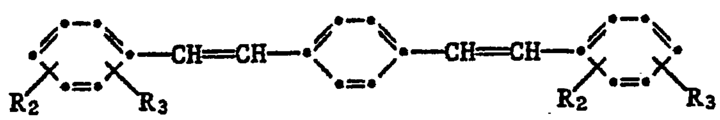
(2)

und/oder

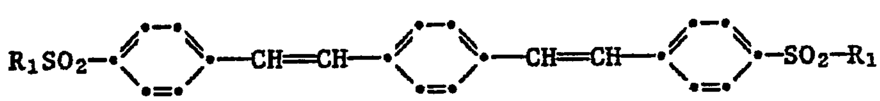
(3)

worin $R_1$ $C_1$-$C_4$-Alkyl oder Phenyl, $R_2$ Cyano, $COOC_1$-$C_4$-Alkyl oder Chlor und $R_3$ Wasserstoff oder Chlor bedeuten.

7. Mittel gemäss Anspruch 6, enthaltend mindestens eine 1,4-Distyrylbenzolverbindung der Formel 1 oder deren Mischungen mit mindestens einer der Verbindungen der Formeln 2 und/oder 3 und eine oder mehrere optische Aufheller aus der Reihe der 1,4-Distyrylbenzole, 4,4'-Distyryl-biphenyle, 2,5-Dibenzoxazolylthiophene, 1,2-Dibenzoxazolyl-äthylene, Mono- und Dibenzoxazolyl-stilbene oder -styrole, Triazinyl-pyrene, 4-Triazolyl-stilbene, Isoxazolyl-stilbene, Dibenzoxazolyl-naphthaline, Triazolyl-, Pyrazolyl- und Phenyl-cumarine, 4,4'-Divinyl-stilbene und Naphthalimide.

8. Verfahren zur Herstellung von 1,4-Distyrylbenzolverbindungen der Formel

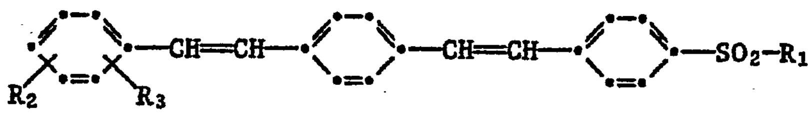

(1)

oder deren Mischungen mit Verbindungen der Formeln

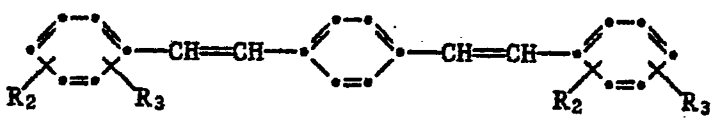

(2)

und/oder

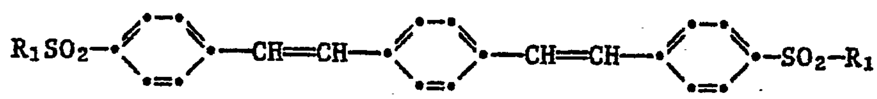

(3)

worin $R_1$ $C_1$-$C_4$-Alkyl oder Phenyl, $R_2$ Cyano, $COOC_1$-$C_4$-Alkyl oder Chlor und $R_3$ Wasserstoff oder Chlor bedeuten, dadurch gekennzeichnet, dass man Terephthalaldehyd in einer oder zwei Reaktionsstufen mit den beiden Phosphonaten der Formeln

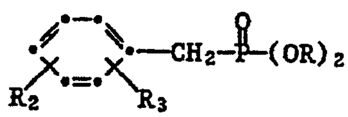

(4)

und

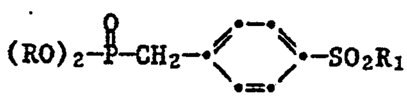

(5)

worin R $C_1$-$C_4$-Alkyl bedeutet, kondensiert.

9. Verfahren gemäss Anspruch 8, dadurch gekennzeichnet, dass man bei der zweistufigen Reaktion die intermediär gebildeten Aldehyde der Formeln

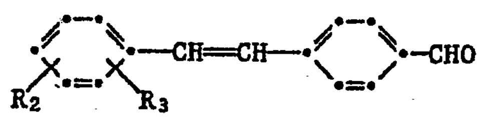

(6)

oder

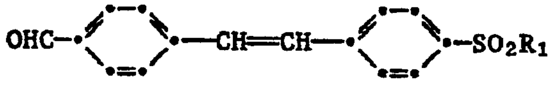

(7)

isoliert.

10. Verfahren gemäss Anspruch 8, dadurch gekennzeichnet, dass man zur Herstellung von Mischungen der Verbindungen der Formel 1 mit Verbindungen der Formel 2 und/oder 3, Terephthalaldehyd mit den beiden Phosphonaten der Formeln 4 und 5 in den jeweils für die Mischungen vorgesehenen stöchiometrischen Mengenverhältnissen kondensiert.

11. Aldehyde der Formel

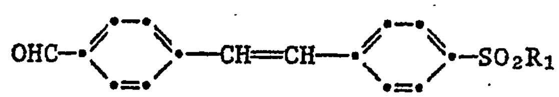

(7)

worin $R_1$ $C_1$-$C_4$-Alkyl oder Phenyl bedeutet.

12. Aldehyd der Formel

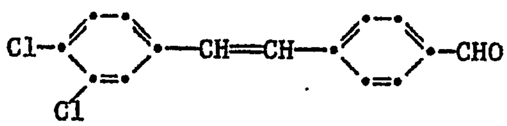

(8)

**Claims**

1. A 1,4-distryrylbenzene compound of the formula

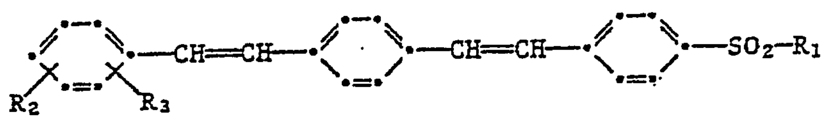

(1)

in which $R_1$ is $C_1$–$C_4$alkyl or phenyl, $R_2$ is cyano, $COOC_1$–$C_4$alkyl or chlorine and $R_3$ is hydrogen or chlorine.

2. A 1,4-distyrylbenzene compound according to claim 1, wherein, in formula 1, $R_1$ is methyl or ethyl, $R_2$ is cyno or 4-chlorine and $R_3$ is hydrogen or, if $R_2$ is 4-chlorine, is 3-chlorine.

3. A 1,4-distyrylbenzene compound according to claim 1, wherein, in formula 1, $R_1$ is methyl, $R_2$ is 2-cyano and $R_3$ is hydrogen.

4. A mixture of compounds of the formula 1 according to claim 1 with compounds of the formulae

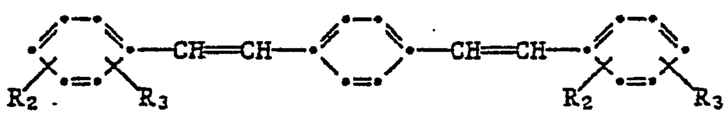

(2)

and/or

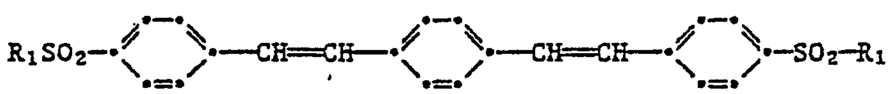

(3)

5. A mixture according to claim 4, containing 1 to 99% by weight of 1-(2-cyanostyryl)-4-(4-methylsulfonylstyryl)–benzene or 1-(2-cyanostyryl)-4-(4-ethylsulfonylstyryl)–benzene, 1 to 99% by weight of 1,4-di-(2-cyanostyryl)-benzene and 0 to 20% by weight of 1,4–di–(4-methylsulfonyl)–benzene or 1,4–di–(4-ethylsulfonyl)-benzene.

6. An agent for the fluorescent brightening of polyester fibres, containing at least one 1,5-distyrylbenzene compound of the formula

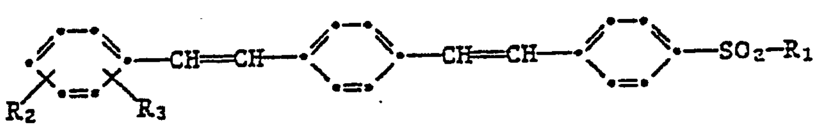

(1)

or mixtures thereof with at least one compound of the formulae

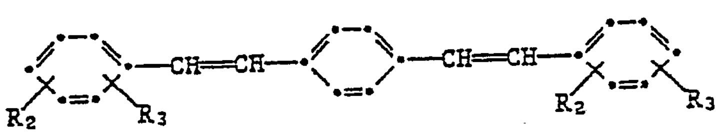

(2)

and/or

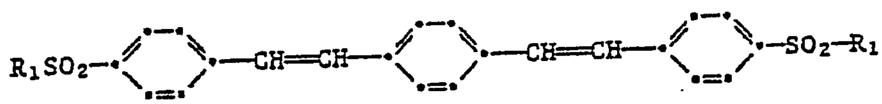

(3)

in which $R_1$ is $C_1$–$C_4$alkyl or phenyl, $R_2$ is cyano, $COOC_1$–$C_4$alkyl or chlorine and $R_3$ is hydrogen or chlorine.

7. An agent according to claim 6, containing at least one 1,4-distyrylbenzene compound of the formula 1 or mixtures thereof with at least one of the compounds of the formulae 2 and/or 3 and one or more fluorescent brighteners belonging to the series comprising 1,4-distyrylbenzenes, 4,4'-distyrylbiphenyls, 2,5-dibenzoxazolylthiophenes, 1,2-dibenzoxazolylethylenes, monobenzoxyzolylstilbenes, monobenzoxazolylstyrenes, dibenzoxazolylstilbenes, dibenzoxazolyllstyrenes, triazinylpyrenes, 4-triazolylstilbenes, isoxazolylstilbenes, dibenzoxazolylnaphthalenes, triazolylcoumarins, pyrazolyl-coumarins, phenylcoumarins, 4,4'-divinylstilbenes and naphtalimides.

8. A process for the preparation of 1,4-distyrylbenzene compounds of the formula

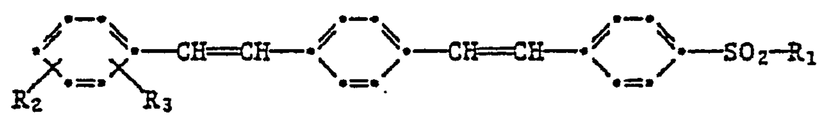

(1)

or mixtures thereof with compounds of the formulae

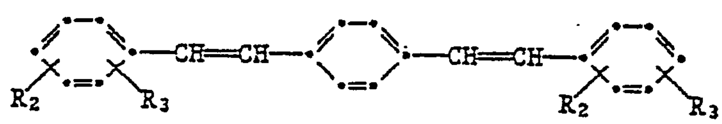

(2)

and/or

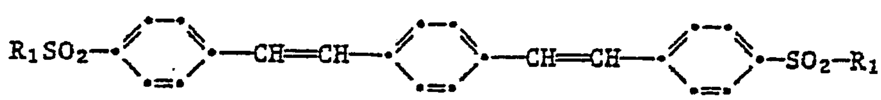

(3)

in which $R_1$ is $C_1$–$C_4$ alkyl or phenyl, $R_2$ is cyano, $COOC_1$–$C_4$alkyl or chlorine, which comprises subjecting terephthalaldehyde to a condensation reaction in one or two reaction stages with the two phosphonates of the formulae

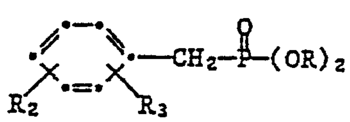

(4)

and

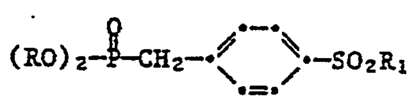

(5)

in which R is $C_1$–$C_4$alkyl.

9. A process according to claim 8, wherein the aldehydes of the formulae

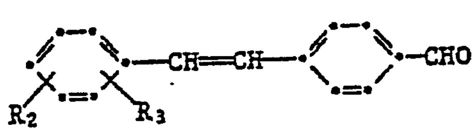

or

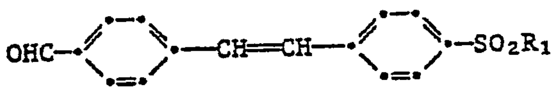 (7)

which are formed as intermediates in the two-stage reaction are isolated.

10. A process according to claim 8, wherein, in order to prepare mixtures of the compounds of the formula 1 with compounds of the formula 2 and/or 3, terephthalaldehyde is subjected to a condensation reaction with the two phosphonates of the formulae 4 and 5 in the stoichiometric with the two phosphonates of the formulae 4 and 5 in the stoichiometric ratios intended for each of the mixtures.

11. An aldehyde of the formula

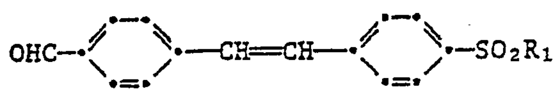 . (7)

in which $R_1$ is $C_1$–$C_4$alkyl or phenyl.

12. An aldehyde of the formula

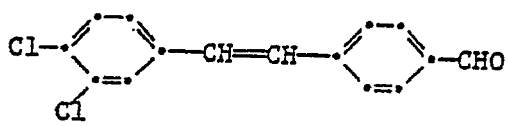 (8)

**Revendications**

1. Composés 1,4-distyrylbenzène de formule:

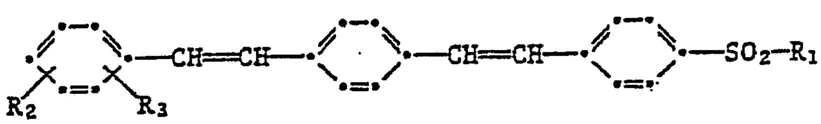 (1)

dans laquelle $R_1$ désigne un gorupe alkyle en $C_1$ à $C_4$ ou un groupe phényle, $R_2$ désigne un groupe cyano, un groupe COO-alkyle($C_1$–$C_4$) ou un atome de chlore et $R_3$ désigne un atome d'hydrogène ou de chlore.

2. Composés 1,4-distyrylbenzène selon la revendication 1, pour lesquels, dans la formule 1, $R_1$ représente un groupe méthyle ou éthyle, $R_2$ représente un groupe cyano ou 4-chloro et $R_3$ représente un atome d'hydrogène ou, lorsque $R_2$ est un groupe 4-chloro, un groupe 3-chloro.

3. Composés 1,4-distyrylbenzène selon la revendication 1, pour lesquels, dans la formule 1, $R_1$ représente un groupe méthyle, $R_2$ représente un groupe 2-cyano et $R_3$ représente un atome d'hydrogène.

4. Mélanges de composés de formule 1 selon la revendication 1 avec des composés de formules:

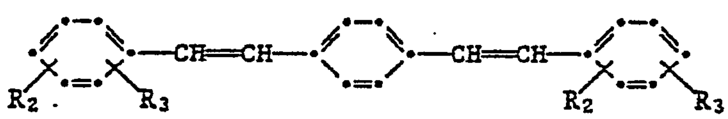 (2)

et/ou

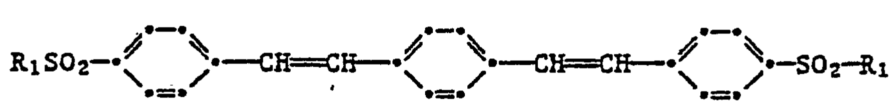 (3)

5. Mélanges selon la revendication 4, contenant 1 à 99% en poids de 1-(2-cyanostyryl)-4-(4-méthylsulfonyl- ou 4-éthylsulfonylstyryl)-benzène, 1 à 99% en poids de 1,4-di(2-cyanostyryl)-benzène et 0 à 20% en poids de 1,4-di(4-méthylsulfonyl- ou 4-éthylsulfonyl-styryl)-benzène.

6. Agent pour l'azurage optique des fibres de polyester, contenant au moins un composé 1,4-distyrylbenzène de formule:

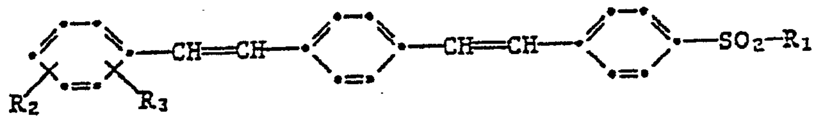

(1)

ou son mélange avec au moins un composé de formules:

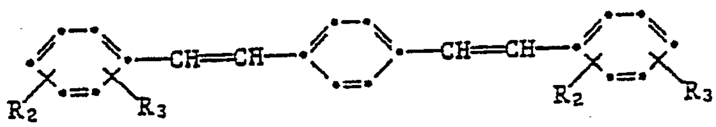

(2)

et/ou

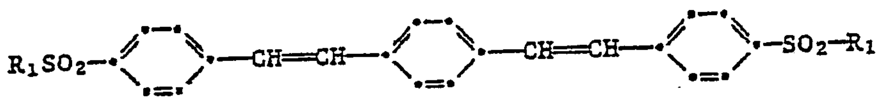

(3)

$R_1$ désignant un groupe alkyle en $C_1$ à $C_4$ ou un groupe phényle, $R_2$ désignant un groupe cyano, un groupe COO-alkyle($C_1$–$C_4$) ou un atome de chlore et $R_3$ désignant un atome d'hydrogène ou de chlore.

7. Agent selon la revendication 6, contenant au moins un composé 1,4-distyrylbenzène de formule 1 ou son mélange avec au moins un des composés de formules 2 et/ou 3, et un ou plusieurs azurants optiques appartenant aux séries de 1,4-distyrylbenzène, 4,4'-distyrylbiphényl, 2,5-dibenzoxazolylthiophène, 1,2-dibenzoxazolyléthylène, mono- et dibenzoxazolylstilbène ou -styrène, triazinylpyrène, 4-triazolylstilbène, isoxazolylstilbène, dibenzoxazolylnaphtalène, triazolyl-, pyrazolyl- et phénylcoumarine, 4,4-divinylstilbène et naphtalimide.

8. Procédé de préparation des composés 1,4-di-styrylbenzène de formule:

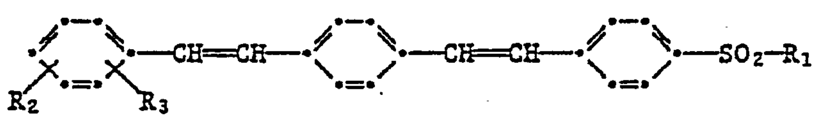

(1)

ou de leurs mélanges avec des composés de formules:

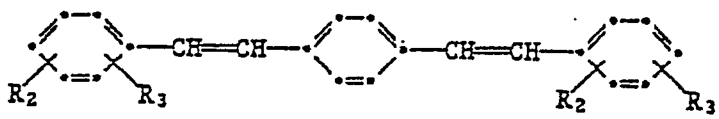

(2)

et/ou

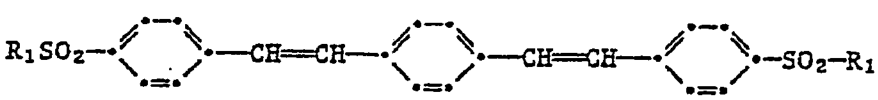

(3)

dans lesquelles $R_1$ représente un groupe alkyle en $C_1$ à $C_4$ ou un groupe phényle, $R_2$ représente un groupe cyano, un groupe COO-alkyle($C_1$–$C_4$) ou un atome de chlore et $R_3$ représente un atome d'hydrogène ou de chlore, caractérisé en ce que l'on condense l'aldéhyde téréphtalique, dans une réaction en une ou deux étapes, avec les deux phosphonates de formules:

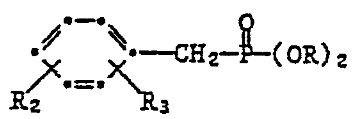

(4)

et

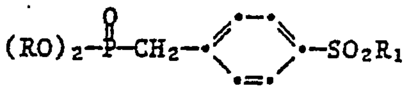

(5)

dans lesquelles R désigne un groupe alkyle en $C_1$ à $C_4$.

9. Procédé selon la revendication 8, caractérisé en ce qu'on isole, dans la réaction en deux étapes, les aldéhydes intermédiairement formés de formules:

(6)

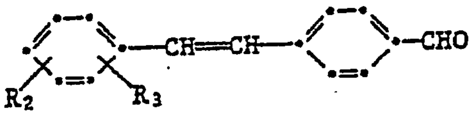

ou

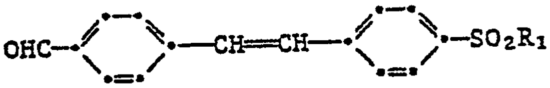

(7)

10. Procédé selon la revendication 8, caractérisé en ce que, pour la préparation de mélanges de composés de formule 1 avec des composés de formules 2 et/ou 3, on condense l'aldéhyde téréphtalique avec les deux phosphonates de formuels 4 et 5 dans les proportions stoechiométriques prévues à chaque fois pour les mélanges.

11. Aldéhyde de formule:

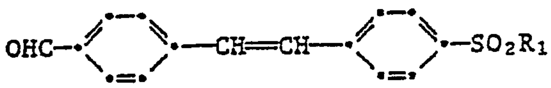

(7)

dans laquelle $R_1$ représente un groupe alkyle en $C_1$ à $C_4$ ou un groupe phényle.

12. Aldéhyde de formule:

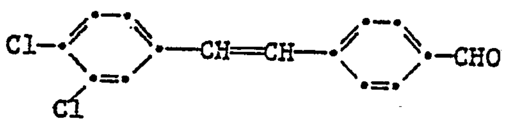

(8)